# EUROPÄISCHE PATENTSCHRIFT

(11) **EP 2 313 117 B1**
(45) Veröffentlichungstag und Bekanntmachung des Hinweises auf die Patenterteilung: **22.02.2012**
(21) Anmeldenummer: 09777609.0
(22) Anmeldetag: 03.08.2009
(51) Int. Cl.: A61L 2/24

(54) **VORRICHTUNG UND VERFAHREN ZUR DETEKTION VON GLASBRUCH IN EINEM DURCHLAUF-STERILISIERTUNNEL**
DEVICE AND METHOD FOR DETECTING BROKEN GLASS IN A CONVEYOR STERILIZATION TUNNEL
DISPOSITIF ET PROCÉDÉ POUR DÉTECTER LA BRISURE DE VERRE DANS UN TUNNEL DE STÉRILISATION CONTINUE

(30) Priorität: 04.08.2008 DE 102008036069
(43) Veröffentlichungstag der Anmeldung: 27.04.2011
(73) Patentinhaber: CSL Behring GmbH, 35041 Marburg (DE); ACCURRO GmbH, 35041 Marburg (DE)
(72) Erfinder: HOBERG, Karl, 35043 Marburg (DE)
(74) Vertreter: Binsack, Beate
(86) Internationale Anmeldenummer: PCT/EP2009/005601
(87) Internationale Veröffentlichungsnummer: WO 2010/015369

(56) Entgegenhaltungen:
- WO-A-2006/029083
- GB-A- 1 349 677
- US-A- 3 527 017

## Beschreibung

Die vorliegende Erfindung betrifft eine Vorrichtung sowie ein Verfahren zur Detektion von Glasbruch in einem zum Sterilisieren oder Depyrogenisieren von Glasbehältern vorgesehenen Durchlaufofen, welcher unter anderem eine Transporteinrichtung für die Glasbehälter aufweist.

### Stand der Technik

Sterilisier- oder Depyrogenisiereinrichtungen in Form eines so genannten Sterilisiertunnels sind als solche aus dem Stand der Technik bekannt. Sie dienen überwiegend dem Sterilisieren bzw. Depyrogenisieren von im pharmazeutischen Bereich zu verwendenden Behältern, insbesondere von Ampullen, Karpulen oder von Behältern aus Röhrenglas, so genannten Vials.

Derartige Sterilisiertunnel sind überwiegend nach dem Durchlaufprinzip ausgebildet, wobei die zu sterilisierenden oder zu depyrogenisierenden Glasbehälter mittels einer, typischerweise als Transportband ausgebildeten Transporteinrichtung durch verschiedene Temperaturzonen innerhalb des Durchlaufofens transportiert werden.

Ein solcher Durchlaufofen ist beispielsweise in der DE 42 17 054 A1 gezeigt, welcher ein elektrisches Heizregister umfasst, über das ein Gebläse die auf Sterilisiertemperatur von beispielsweise 320° C erhitzte Luft in einem Umluft-Kreislauf zirkulieren lässt. Dieser Umluft-Kreislauf führt dabei von dem Gebläse über heißluftbeständige Hochleistungs-Schwebstofffilter von oben nach unten durch den Sterilisierkanal, in welchem auch die Heizregister angeordnet sind.

Um ein ausreichendes Sterilisieren bzw. Depyrogenisieren erreichen zu können, müssen die zu sterilisierenden bzw. depyrogenisierenden Glasbehälter über eine gewisse Zeitdauer, welche typischerweise im Minutenbereich liegt, der vorgegebenen Sterilisier- bzw. der Depyrogenisiertemperatur ausgesetzt sein. Hiernach werden die Behälter auf ein niedrigeres Temperaturniveau abgekühlt, bei welcher ein Befüllen und Verschließen der Behälter erfolgen kann. Das Aufheizen bzw. Abkühlen der Behälter erfolgt unter einem verhältnismäßig großen zeitlichen Temperaturgradienten. So kann die Aufheizung auf die Sterilisier- oder Depyrogenisiertemperatur innerhalb einer Minute oder gar in einem deutlich kürzeren Zeitintervall erfolgen.

Aufgrund dieser systembedingten Temperaturschwankungen beim Durchfahren durch einen solchen Sterilisier- oder Depyrogenisiertunnel, aber auch bedingt durch eine dichte, gegebenenfalls sogar gedrängte Anordnung der Glasbehälter auf der durch den Sterilisier- bzw. Depyrogenisiertunnel führenden Transporteinrichtung kann vereinzelt Glasbruch auftreten, welcher mit einer Bildung von kleinsten Glaspartikeln oder Glasscherben einhergeht.

Dies ist nicht nur im Hinblick auf den jeweils zerstörten oder beschädigten Glasbehälter von Nachteil, sondern es tritt insbesondere bei einem durch thermische Spannungen im Material induzierten Zerbersten eines Glasbehälters das Problem auf, dass die auf der Transporteinrichtung nach oben offen ausgebildeten und in unmittelbarer Umgebung zum zerborstenen Glasbehälter befindlichen Glasbehälter mit Glaspartikeln kontaminiert werden.

Das Entfernen solcher Glaspartikel oder Glasscherben aus den betroffenen Glasbehältern ist äußerst aufwändig. Zwar existieren Lösungsansätze, die Behälter umzudrehen, sie sozusagen auf den Kopf zu stellen und mit Luft auszublasen, um etwaige darin anhaftende Scherben oder Glaspartikel zu entfernen. Da die zur Aufnahme pharmazeutischer Substanzen ausgebildeten Glasbehälter jedoch ohnehin als Wegwerf- und Einmalartikel vorgesehen sind, ist ein solches Verfahren zum Entfernen von Glasscherben oder Glaspartikeln wirtschaftlich unrentabel.

Auf jeden Fall sollte aber vermieden werden, dass ein mittels Glasbruch entstandenen Glaspartikeln kontaminierter Glasbehälter mit einer pharmazeutischen Substanz, etwa mit einem flüssigen Medikament oder einem Impfstoff gefüllt wird, da der Wert der in einem einzigen Glasbehälter aufnehmbaren pharmazeutischen Substanz das bis zu 100- oder 1000-fache der Herstellungskosten eines Glasbehälters übersteigen kann.

Die US 3,527,017 beschreibt ein automatisches System zum Abfüllen von Containern, wie z.B. Vials, bei dem die Fördereinrichtung Mittel vorsieht, die auf die Seite gefallen sind, zu entfernen. Ferner sieht die US 3,527,017 eine Lichtschranke in der Ebene der Fördereinrichtung vor die dazu dient die Vials zu zählen, bis die Anzahl mit der der Verpackungsgröße übereinstimmt.

### Aufgabe

Der vorliegenden Erfindung liegt daher die Aufgabe zugrunde, ein möglichst wirtschaftliches, einfach zu implementierendes, besonders einfach zu bedienendes und obendrein zuverlässiges System zur Detektion von Glasbruch in einem Sterilisier- oder Depyrogenisiertunnel zur Verfügung zu stellen.

### Erfindung und vorteilhafte Wirkungen

Die der Erfindung zugrunde liegende Aufgabe wird mittels einer Detektionsvorrichtung gemäß Patentanspruch 1 und einem Verfahren zur Detektion von Glasbruch gemäß Patentanspruch 14 gelöst. Vorteilhafte Ausführungsformen der Erfindung sind in den jeweiligen abhängigen Patentansprüchen angegeben.

Die erfindungsgemäße Vorrichtung ist zur Detektion von Glasbruch in einem zum Sterilisieren oder Depyrogenisieren von Glasbehältern vorgesehenen Durchlaufofen ausgebildet, wobei der Durchlaufofen zumindest eine Transporteinrichtung für die Glasbehälter aufweist, mittels welcher die Glasbehälter durch den Durchlaufofen hindurchgeführt werden können. Der Durchlaufofen weist zumindest einen solchen Temperaturbereich auf, bei welchem so genannte Pyrogene, das heißt entzündlich wirkende Abbauprodukte und Zellrestbestandteile von abgetöteten Keimen vollständig entfernt werden.

Die erfindungsgemäße Detektionsvorrichtung zeichnet sich durch zumindest eine Sende- und Empfangseinheit aus, wobei die Empfangseinheit zumindest zur Detektion solcher elektromagnetischer Strahlen ausgebildet ist, welche von der Sendeinheit emittiert werden. Dabei ist ferner vorgesehen, dass die Transporteinrichtung des Durchlaufofens und die Sende- und Empfangseinheit derart zueinander positioniert und/oder derart zueinander ausgerichtet sind, dass infolge von Glasbruch erzeugte Glaspartikel oder Glasscherben von beschädigten oder zerborstenen Glasbehältern unmittelbar bei einem oder nach einem Verlassen einer von der Transporteinrichtung vorgegebenen im Wesentlichen horizontal verlaufenden Transportebene den Strahlengang zwischen Sende- und Empfangseinheit kreuzen.

Die Bewegung der durch Glasbruch erzeugten Glaspartikel aus der Transportebene heraus erfolgt vorzugsweise schwerkraftbedingt. Sobald ein Glaspartikel den Strahlengang zwischen Sende- und Empfangseinheit kreuzt wird der Strahlengang zumindest für eine kurze Zeitdauer unterbrochen, zumindest aber abgeschwächt oder in einer von der Empfangseinheit detektierbaren Art und Weise verändert. Die den Strahlengang zwischen Sende- und Empfangseinheit kreuzenden Glaspartikel sind dazu ausgebildet, den Strahlengang durch Brechung und/oder Reflexion zu verändern und/oder die an der als Detektor ausgebildeten Empfangseinheit detektierbare Strahlungs-Intensität zu verändern.

Die erfindungsgemäße Vorrichtung und das mit ihr zu verwirklichende Detektionsverfahren beruht auf der Erkenntnis, dass beim Zerbersten oder Brechen eines Glasbehälters, etwa einer Ampulle, einer Karpule, Septengläsern, Spritzen , Vials oder sonstigen beim Zerbrechen so genannter Parenteralia-Verpackungen entstehenden Glasscherben in Zwischenräumen zwischen den Glasbehältern auf der Transporteinrichtung zu liegen kommen. Da die Transporteinrichtung typischerweise als umlaufendes Transportband ausgebildet ist, fallen die auf dem Band infolge von Glasbruch erzeugten Glaspartikel an einem Umlenkpunkt des Transportbandes, typischerweise am Ende eines Transportwegs von dem Band herunter und werden somit aus der vom Transportband vorgegebenen Transportebene heraus befördert.

Durch entsprechende Anordnung der Transporteinrichtung und der Sende- und Empfangseinheit zueinander kann ein Herunterfallen von Glaspartikeln von der Transporteinrichtung berührungslos detektiert werden. Infolge einer solchen Detektion, welche mittels einer akustischen oder optischen Signaleinrichtung für das Bedienpersonal des Durchlaufofens bzw. der Transporteinrichtung erkennbar gemacht werden kann, ist nach der Erfindung vorgesehen, eine vorgegebene Anzahl in unmittelbarer Nähe zum zerborstenen und detektierten Behälter angeordneter Behälter prophylaktisch aus dem Produktions- Reinigungs- oder Abfüllzyklus zu entfernen.

Dabei ist insbesondere vorgesehen, auch solche Behälter, welche bei Detektion von Glasbruch die Transporteinrichtung bereits verlassen haben, prophylaktisch aus einer dem Durchlaufofen nachgeschalteten Prozessstation zu entnehmen, damit hinreichend sichergestellt werden kann, dass sämtliche potentiell mit Glaspartikeln kontaminierten und aufgrund des Zerbrechens eines einzigen Glasbehälters zum Zeitpunkt des Zerbrechens um diesen herum platzierten Glasbehälter aus dem Produktionsablauf entfernt sind.

Zwar wird auf diese Art und Weise womöglich eine Vielzahl von Glasbehälter bei Auftreten eines einzigen Glasbruchs prophylaktisch vernichtet und/oder einem Recyclingkreislauf zugeführt. Aufgrund der Tatsache, dass mit diesem Verfahren Glasbruch besonders zuverlässig und hinreichend sicher detektiert werden kann und in den laufenden Produktionsprozess lediglich bei Auftreten eines tatsächlich erfolgten Glasbruchs manuell eingegriffen werden muss, gestaltet sich das mit der vorliegenden Detektionsvorrichtung durchführbare Detektionsverfahren als besonders fertigungsrationell.

Insbesondere in Anbetracht der Herstellungskosten für ein einziges Glasbehältnis und der Häufigkeit auftretenden Glasbruchs, welcher mit deutlich weniger als 1 Promille angegeben werden kann, fällt ein prophylaktisches Herausnehmen einer Anzahl von Glasbehältern um einen zerborstenen Glasbehälter herum nicht sonderlich ins Gewicht.

Nach einer vorteilhaften Ausführung der Erfindung ist vorgesehen, dass die zwischen Sendeeinheit und Empfangseinheit propagierenden Strahlen, insbesondere Lichtstrahlen im sichtbaren, aber auch im infraroten oder ultravioletten Spektralbereich, im Wesentlichen senkrecht zur Bewegungsrichtung der Glaspartikel oder der Glasscherben verlaufen. Mit Bewegungsrichtung der Glaspartikel oder Glasscherben ist insbesondere diejenige Bewegungsrichtung der Glaspartikel gemeint, welche diese nach Verlassen der Transporteinrichtung einnehmen.

Besonders vorteilhaft gestaltet sich die Anordnung von Sendeeinheit und Empfangseinheit unterhalb der Transporteinrichtung, insbesondere im Bereich eines in Förderrichtung vorn liegenden Endabschnitts der Transporteinrichtung. Auf diese Art und Weise können diejenigen, auf der Transporteinrichtung zu liegen kommenden und infolge von Glasbruch entstehenden Glaspartikel oder Glasscherben unmittelbar beim Herunterfallen von der Detektionseinrichtung etwa im Bereich des Umlenkpunkts einer als Transportband ausgebildeten Transporteinrichtung detektiert werden.

Dabei ist ferner vorgesehen, dass der Abstand zwischen Sendeeinheit und Empfangseinheit zumindest der Breite des Transportbandes entspricht, sodass sämtliche auf dem Transportband liegenden Glaspartikel unabhängig von ihrer Anordnung quer zur Transportrichtung von der Empfangseinheit beim Durchkreuzen des Strahlengangs detektiert werden können.

Nach einer weiteren besonders vorteilhaften Ausführungsform der Erfindung ist vorgesehen, dass unterhalb und in Bezug zu einer in Transportrichtung der Transporteinrichtung liegenden vorderen Kante eine Auffangeinrichtung für Glaspartikel oder Glasscherben angeordnet ist. Diese Auffangeinrichtung kann insbesondere trichterförmig ausgebildet sein und sowohl in Förderrichtung als auch schräg oder quer hierzu eine solche Erstreckung oder einen entsprechenden Trichterquerschnitt aufweisen, die hinreichend gewährleisten, dass sämtliche mittels der Transporteinrichtung beförderten Glaspartikel oder Glasscherben in der Auffangeinrichtung landen.

Nach einer bevorzugten Weiterbildung ist dabei vorgesehen, dass die Auffangeinrichtung zumindest eine der Breite der Transporteinrichtung entsprechende Erstreckung aufweist oder zumindest abschnittsweise trichterartig verjüngend ausgebildet ist und/oder mit einem Auslauf in eine Zuführeinrichtung für die Sende- und Empfangseinheit mündet.

Diese Zuführeinrichtung ist vorzugsweise als schiefe Ebene ausgebildet und soll ein Herabgleiten der von der Auffangeinrichtung aufgenommenen und an die Zuführeinrichtung weitergeleiteten Glaspartikel oder Glasscherben ermöglichen. Die Zuführeinrichtung stellt aufgrund ihrer sowohl von der senkrechten als auch waagerechten abweichenden Neigung eine definierte Ebene dar, in welcher die an der Zuführeinrichtung entlang gleitenden oder an ihr herabrutschenden Glaspartikel oder Glasscherben mittels der Sende- und Empfangseinheit sicher und zuverlässig ermittelt werden können.

Dabei ist ferner vorgesehen, dass die Zuführeinrichtung, bzw. ihre schiefe Ebene zumindest bezüglich ihres Neigungswinkels verstellbar ausgebildet ist. Auf diese Art und Weise kann die Geschwindigkeit der entlang der Zuführeinrichtung herabgleitenden Glaspartikel gezielt beeinflusst und gesteuert werden. Am Fuße oder an einem unteren Abschnitt der Zuführeinrichtung sind dabei die Sende- und Empfangseinheit angeordnet, wobei der zwischen Sende- und Empfangseinheit propagierende Strahl elektromagnetischer Strahlung im Wesentlichen in der schiefen Ebene der Zuführeinrichtung und senkrecht zu deren Neigungsrichtung verläuft.

Daneben kann auch vorgesehen sein, die Ausrichtung bzw. die Horizontalkomponente der Zuführeinrichtung und ihrer schiefen Ebene in Bezug auf die von der Transporteinrichtung vorgegebene Förderrichtung veränderlich auszugestalten. Auf diese Art und Weise kann etwa den Bauraum- oder Platzanforderungen im Bereich des Durchlaufofens und/oder nachgeschalteter Bearbeitungsstationen universell Rechnung getragen werden. Dabei ist beispielsweise denkbar, die schiefe Ebene als ein Führungsblech auszubilden, welches an einem schwenkbaren Ständer unterhalb eines Auslaufs einer trichterförmig ausgebildeten Auffangeinrichtung angeordnet ist.

Von Vorteil ist die Zuführeinrichtung zur Sende- und Empfangseinheit hin verjüngend ausgebildet, sodass der Abstand zwischen Sende- und Empfangseinheit entsprechend kurz, insbesondere kleiner als die Breite der Transporteinrichtung gewählt werden kann. Durch die verhältnismäßig dichte Anordnung von Sende-und Empfangseinheit zueinander können solche, eine Detektion beeinflussenden Störungen auf ein Minimum reduziert werden. Auch wird hierdurch die gesamte Detektionsvorrichtung unempfindlicher gegenüber mechanischen Erschütterungen oder Vibrationen.

Des Weiteren ist nach der Erfindung vorgesehen, dass die Sende- und Empfangseinheit an zueinander gegenüberliegenden Seitenrändern der Transporteinrichtung oder der Zuführeinrichtung angeordnet sind. Auf diese Art und Weise kann erreicht werden, dass die zwischen Sende- und Empfangseinheit verlaufenden Strahlen in der Ebene der Transporteinrichtung und/oder in der Ebene der Zuführeinrichtung propagieren oder jeweils im Wesentlichen parallel hierzu verlaufen.

Insbesondere kann hierbei vorgesehen sein, dass Sende- und Empfangseinheit derart zum Transportband angeordnet oder ausgerichtet sind, dass die zwischen Sende- und Empfangseinheit propagierenden Strahlen sowohl im Wesentlichen senkrecht zur Transportrichtung des Transportbandes als auch im Wesentlichen senkrecht zur Flächennormalen des Transportbandes verlaufen. Die Strahlen überstreichen somit vorzugsweise die gesamte Breite des Transportbandes bzw. der dem Transportband nachgeschalteten Zuführeinrichtung, sodass sämtlicher erzeugter Glasbruch sicher und zuverlässig detektiert werden kann.

Des Weiteren ist vorgesehen, dass die Sendeeinheit als monochromatische Lichtquelle, etwa als Laserlichtquelle ausgebildet ist. Diese kann entweder in einem gepulsten oder in einem so genannten Continnous Wave Modus also mit einem kontinuierlich lichtemittierenden Strahl betrieben werden. Im gepulsten Modus ist insbesondere darauf zu achten, dass die Pulsfrequenz derart hoch ist, dass das Zeitintervall zweier aufeinander folgender Pulse kleiner als dasjenige Zeitintervall ist, welches von einem Glaspartikel benötigt wird, um den Strahlengang zwischen Sende- und Empfangseinheit zu durchkreuzen.

Unabhängig von der Betriebsweise der Sendeeinheit ist ferner darauf zu achten, dass die Abtastrate der Detektionseinheit, welche im einfachsten Fall als Photodiode ausgebildet sein kann, der Geschwindigkeit und der Größe der Glaspartikel gerecht wird, so dass keine Glaspartikel undetektiert an der Detektionseinrichtung vorbeirutschen.

Nach einer Weiterbildung oder alternativen Ausgestaltung der Erfindung kann auch vorgesehen sein, die Transporteinrichtung als ein mit einem Gittergeflecht versehenes Transportband mit einer Vielzahl von Öffnungen auszubilden und mehrere Sende- und Empfangseinheiten paarweise und in Förderrichtung von einander beabstandet entlang der von der Transporteinrichtung zu überwindenden Verfahrstrecke anzuordnen. Dabei kann insbesondere vorgesehen sein, unterhalb des in Förderrichtung laufenden Transportbandes mittels einer entsprechenden Optik oder mittels mehrerer Sende- und Empfangseinheiten eine Art Licht- oder Strahlungsebene auszubilden, welche dazu vorgesehen ist, unmittelbar bei Auftreten von Glasbruch die durch die Zwischenräume des Transportbandes gelangenden Glaspartikel zu detektieren.

Auf diese Art und Weise kann entstehender Glasbruch nahezu ohne Zeitverzögerung detektiert werden. Mittels einer solchen Fördereinrichtung und der Vielzahl einzelner Sende- und Empfangseinrichtungen kann beispielsweise auch der Ort des erfolgten Glasbruchs präzise ermittelt werden, sodass ein manuelles Eingreifen zum Entfernen und Herausnehmen einer Anzahl von möglicherweise mit Glaspartikeln kontaminierten Behälter bereits vor einem Herabfallen von Glaspartikeln am Ende der Transporteinrichtung erfolgen kann.

Mit der Erfindung ist ferner vorgesehen, dass die Empfangseinheit an zumindest eine Auswerteeinheit gekoppelt ist, welche zur Erzeugung eines akustisch und/oder optisch wahrnehmbaren Warnsignals ausgebildet ist. Zur Erzeugung eines optisch wahrnehmbaren Warnsignals kommt beispielsweise eine Blink- oder Warnleuchte in Frage, während als akustischer Signalgeber eine Hupe, Sirene oder ein vergleichbares Alarm erzeugendes Instrument Verwendung finden kann.

Dabei ist ferner vorgesehen, dass die Auswerteeinheit an eine Steuereinheit des Durchlaufofens und/oder der Fördereinrichtung gekoppelt ist, sodass unmittelbar bei oder nach Detektion eines Glasbruchsereignisses der Durchlaufofen, bzw. seine Fördereinrichtung selbsttätig abgeschaltet oder angehalten werden kann.

Nach einem weiteren unabhängigen Aspekt betrifft die Erfindung ein Verfahren zur Detektion von Glasbruch in oder an einem zum Sterilisieren oder Depyrogenisieren von Glasbehältern vorgesehenen Durchlaufofen, wobei der Durchlaufofen zumindest eine Transporteinrichtung für die Glasbehälter aufweist, mittels welcher die Glasbehälter durch den Durchlaufofen hindurchgeführt werden. Dabei ist ferner vorgesehen, zumindest einige, bevorzugt aber sämtliche Glaspartikel oder Glasscherben, welche eine von der Transporteinrichtung vorgegebene Transportebene verlassen, mittels zwischen einer Sende- und Empfangseinheit propagierender elektromagnetischer Strahlung zu detektieren.

Die Detektion erfolgt dabei während oder nach einem Verlassen eines umlaufenden Transportbandes der Fördereinrichtung. Das detektierte Ereignis wird sodann akustisch und/oder optisch für das Bedienpersonal des Durchlaufofens signalisiert und/oder zum selbsttätigen Anhalten oder Unterbrechen zumindest der Transporteinrichtung verwendet.

### Ausführungsbeispiele

Weitere Ziele, Merkmale sowie vorteilhafte Anwendungsmöglichkeiten der Erfindung gehen aus der nachfolgenden Beschreibung von Ausführungsbeispielen hervor. Dabei bilden sämtliche wörtlich vorher und im Folgenden beschriebenen als auch in den Figuren bildlich dargestellten Merkmale in jeglicher sinnvollen Kombination untereinander den Gegenstand der vorliegenden Erfindung.

Es zeigen:
- Figur 1: eine schematische perspektivische Darstellung der Fördereinrichtung und einer zugeordneten Sende- und Empfangseinheit,
- Figur 2: eine schematische Seitenansicht einer weiteren Ausführungsform mit einer Auffangeinrichtung und einer Zuführeinrichtung,
- Figur 3: eine perspektivische schematische Darstellung der Auffang- und Zuführeinrichtung und
- Figur 4: ein Blockdiagramm einer elektrischen Auswerteeinheit und damit gekoppelte Komponenten.

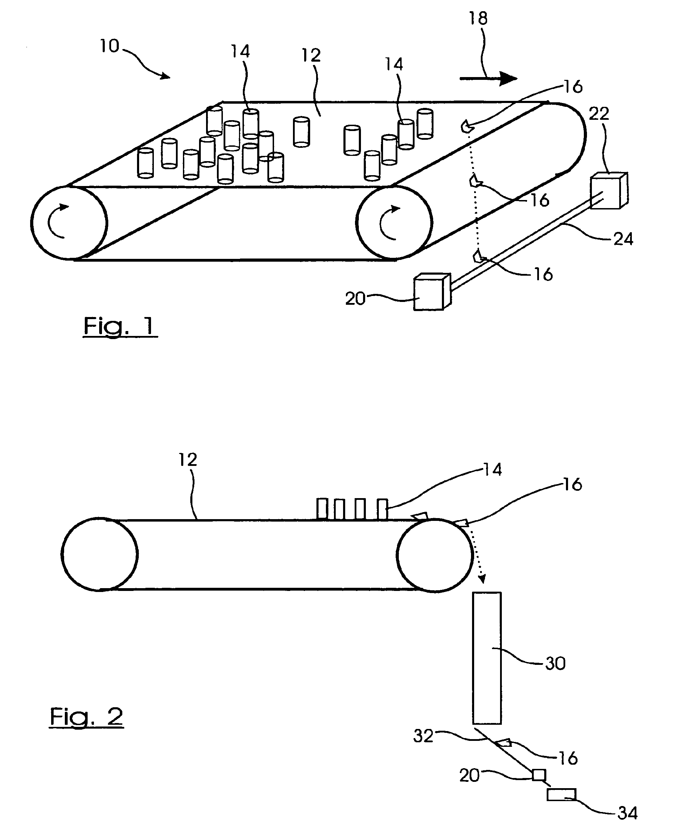

Die in Figur 1 gezeigte Transporteinrichtung 10 weist ein umlaufendes Transportband 12 auf, welches beispielsweise als Gittergeflecht ausgebildet sein kann, um ein vertikales Durchströmen von Heißluft innerhalb des in den Figuren nicht explizit gezeigten Durchlaufofens zu ermöglichen. Das Transportband 12 wird mit einer Vielzahl von Glasbehältern 14, welche nach oben geöffnet sein können, von links beschickt und mittels dem Transportband 12 durch den in den Figuren nicht explizit gezeigten Sterilisier- oder Depyrogenisiertunnel befördert, wobei die Glasbehälter 14 innerhalb kürzester Zeit auf die Sterilisier- oder Depyrogenisiertemperatur, etwa im Bereich von 300° C und darüber hinaus für eine vorgegebene Zeit aufgeheizt werden.

Obschon in Figur 1 Zwischenräume zwischen einzelnen Behältern 14 zu sehen sind, werden die Behälter typischerweise dicht gedrängt zueinander und einander berührend der Transporteinrichtung zugeführt. Das dichte Nebeneinander- oder Aneinanderstellen einzelner, oftmals zylindrisch oder länglich ausgebildeter Glasbehälter 14 verhindert einerseits Umfallen, insbesondere wenn das Transportband 12 in der durch den Pfeil 18 angedeuteten Förderrichtung bewegt wird.

Neben den Glasbehältern 14 können einzelne durch das Gittergeflecht des Transportbandes 12 nicht hindurchfallende Glasscherben oder Glaspartikel 16 welche durch ein Zerbersten oder Zerplatzen eines einzelnen oder mehrerer Glasbehälter 14 in Zwischenräumen zwischen umliegenden Glasbehältern 14 auf dem Transportband 12 liegen bleiben. Diese Glaspartikel 16 werden vom Transportband in Förderrichtung nach rechts bewegt, wo sie am Ende der Verfahrstrecke in Folge der Umlenkung des Transportbandes an einer Umlenkrolle durch die Schwerkraft bedingt nach unten fallen.

Diese Fallbewegung von Glaspartikeln oder Glasscherben 16 kann mittels einer Detektionseinrichtung, welche eine Empfangseinheit 20 und eine zugeordnete Sendeeinheit 22 für elektromagnetische Strahlung 24 aufweist detektiert werden. In einer einfachen Ausgestaltung kann die Detektionsvorrichtung 20, 22 am Ende der Verfahrstrecke des Transportbandes 12 und etwas unterhalb von der rechts, in Transportrichtung liegenden Umlenkrolle angeordnet sein. Prinzipiell genügt es, die Detektionsvorrichtung als Lichtschranke auszubilden, wenngleich die Implementierung einer Laserlichtquelle als Sendeeinheit, insbesondere auf Halbleiterbasis als vorteilhaft anzusehen ist.

Empfangseinheit 20 und Sendeeinheit 22 sind in der Ausgestaltung gemäß Fig. 1 vorzugsweise seitlich des Transportbandes 12 angeordnet, sodass die elektromagnetische Strahlung im Wesentlichen parallel zur Ebene des Transportbandes 12 propagierend sämtlichen vom Transportband 12 herunterfallenden Glasbruch zuverlässig detektieren kann. Sende- und Empfangseinheit 22, 20 sind dabei links und rechts eines unteren Endabschnittes der Zuführeinrichtung 32 angeordnet.

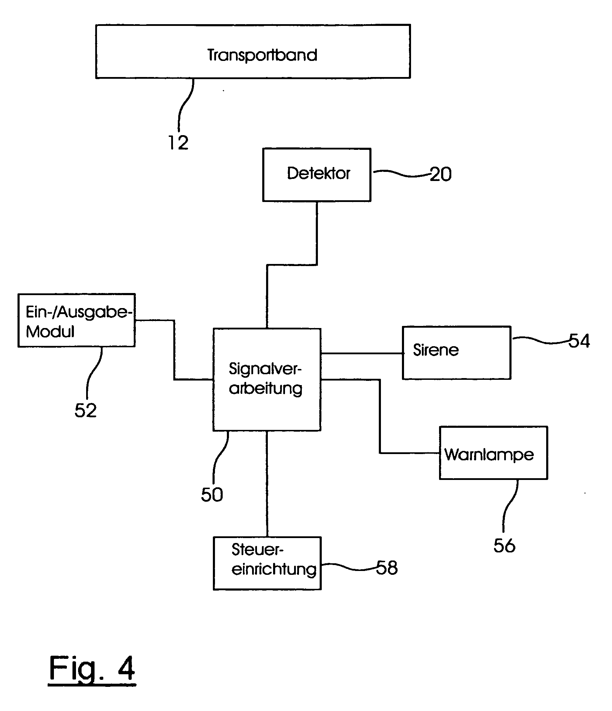

Sobald ein von der Transporteinrichtung 10 herabfallender Glaspartikel 16 den Strahlengang 24 durchkreuzt, kann dieser mittels der Detektoreinheit 20 detektiert und in ein elektrisch auswertbares Signal an eine, in Figur 4 exemplarisch dargestellte Auswerteeinheit 50 weitergegeben werden. Infolge der Detektion und der kurzzeitigen Unterbrechung oder Abschwächung des von der Empfangseinheit 20 detektierbaren Signals kann die Auswerteeinheit mittels eines akustischen Signalgebers 54 und/oder mittels eines optischen Signalgebers 56 das Glasbruchereignis für das Bedienpersonal des Durchlaufofens erkennbar machen.

Daneben kann vorgesehen sein, dass die Auswerteeinheit 50 unmittelbar mit einer Steuereinheit 58 entweder des gesamten Durchlaufofens oder seiner Transporteinrichtung 10 gekoppelt ist, sodass auch ohne Eingreifen von Bedienpersonal das Transportband 12 zur prophylaktischen Entnahme von möglicherweise mit Glaspartikeln kontaminierten Glasbehältern 14 angehalten werden kann. Daneben ist auch denkbar, ohne ein Anhalten der Transporteinrichtung 12 eine vorgegebene Anzahl, etwa einige Reihen von Glasbehältern 14 aus dem Produktionszyklus manuell oder maschinell herauszunehmen.

Daneben ist vorgesehen, dass die Auswerteeinheit 50 mit einem Ein- und Ausgabemodul 52 gekoppelt ist, mittels welchem beispielsweise eine Auslöseschwelle für ein Alarmsignal oder aber die Art und Weise des zu erzeugenden Alarms eingestellt werden kann. Mittels der Ein- und Ausgabeeinrichtung 52 kann ferner die Detektion von fortlaufenden Glasbruchereignissen dokumentiert und ausgewertet werden.

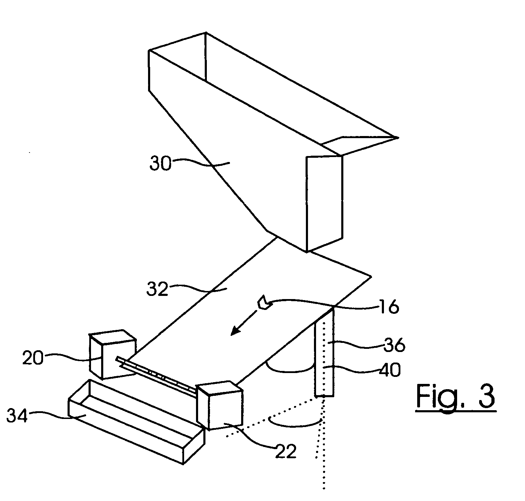

In den Figuren 2 und 3 ist eine weitere Ausführungsform der Erfindung schematisch wiedergegeben. Dabei ist zwischen einer Umlenkrolle des Transportbandes 12 und der Sende- und Empfangseinheit 20, 22 eine nach Art eines Trichters ausgebildete Auffangeinrichtung 30 angeordnet. Dieser ist eine Zuführeinrichtung 32 nachgeschaltet, welche als schiefe Ebene ausgebildet ist, und die die von der Auffangeinrichtung 30 aufgefangenen Glaspartikel in kontrollierter Art und Weise durch die zwischen Sende- und Empfangseinheit 22, 20 gebildete Lichtschranke 24 führt.

Die schiefe Ebene 32 der Zuführeinrichtung kann dabei verstellbar bezüglich ihres Neigungswinkels an einer Halterung 36 angeordnet sein, damit durch Wahl eines geeigneten Neigungswinkels α die Geschwindigkeit oder eine Geschwindigkeitsbandbreite der die Lichtschranke 24 durchkreuzenden Glaspartikel 16 gezielt eingestellt werden kann. Unterhalb und am Ende der schiefen Ebene 32 ist ein Auffangbehälter 34 angeordnet, in welchem die aufgegangenen und mittels der Lichtschranke 24 detektierbaren Glaspartikel oder Glasscherben 16 letztlich aufgefangen werden können.

Neben der Verstellmöglichkeit des Neigungswinkels α kann ferner vorgesehen sein, auch die schiefe Ebene 32 hinsichtlich ihrer Horizontalkomponente variabel auszurichten. So kann insbesondere vorgesehen sein, die gesamte Zuführeinrichtung 32 bezüglich einer Schwenkachse 40 um einen Winkel θ zu drehen, welche vorzugsweise durch den Halter 36 verläuft. Auf diese Art und Weise kann sich die schiefe Ebene 32 bezüglich ihrer Horizontalkomponente etwa in Förderrichtung, entgegen der Förderrichtung oder schräg hierzu, insbesondere senkrecht erstrecken. Auf diese Art und Weise kann die gesamte Detektionsvorrichtung universell an bereits bestehende Transporteinrichtungen und/oder Durchlauföfen gekoppelt und an deren Außen- oder Innenabmessungen individuell angepasst werden.

### Bezugszeichenliste

- 10: Transporteinrichtung
- 12: Transportband
- 14: Glasbehälter
- 16: Glasscherbe
- 18: Pfeil
- 20: Detektor
- 22: Sender
- 24: Strahlengang
- 30: Auffangeinrichtung
- 32: Zuführeinrichtung
- 34: Auffangbehälter
- 36: Halter
- 40: Schwenkachse
- 50: Auswerteeinheit
- 52: Ein-/Ausgabemodul
- 54: akustischer Signalgeber
- 56: optischer Signalgeber
- 58: Steuereinheit

## Patentansprüche

1. Vorrichtung zur Detektion von Glasbruch in einem zum Sterilisieren oder Depyrogenisieren von Glasbehältern (14) vorgesehenen Durchlaufofen, welcher eine Transporteinrichtung (12) für die Glasbehälter aufweist, wobei die Detektionsvorrichtung zumindest eine Sende- und Empfangseinheit (22, 20) umfasst, wobei die Empfangseinheit (20) zur Detektion der von der Sendeeinheit emittierbaren elektromagnetischen Strahlung (24) ausgebildet ist, **dadurch gekennzeichnet, dass** die Transporteinrichtung (12) und die Sende- und Empfangseinheit (22, 20) derart zueinander positioniert und ausgerichtet sind, dass die infolge von Glasbruch erzeugten Glaspartikel oder Glasscherben (16) beim oder nach einen Verlassen einer von der Transporteinrichtung (12) vorgegebenen im Wesentlichen horizontal verlaufenden Transportebene den Strahlengang (24) zwischen Sende- und Empfangseinheit (22, 20) kreuzen und dass die Sendeeinheit (22) und die Empfangseinheit (20) unterhalb der Transporteinrichtung (12) angeordnet sind.

2. Vorrichtung nach Anspruch 1, wobei die zwischen Sendeeinheit (22) und Empfangseinheit (20) propagierenden Strahlen (24) im Wesentlichen senkrecht zur Bewegungsrichtung der Glaspartikel oder Glasscherben (16) verlaufen.

3. Vorrichtung nach einem der vorhergehenden Ansprüche, wobei die Sendeeinheit (22) und die Empfangseinheit (20) im Bereich eines in Förderrichtung (18) vorn liegenden Endabschnitts der Transporteinrichtung (12) angeordnet sind.

4. Vorrichtung nach einem der vorhergehenden Ansprüche, wobei unterhalb und fluchtend zu einer in Transportrichtung liegenden vorderen Kante der Transporteinrichtung (12) eine Auffangeinrichtung (30) für Glaspartikel oder Glasscherben (16) angeordnet ist.

5. Vorrichtung nach Anspruch 4, wobei die Auffangeinrichtung (30) zumindest eine der Breite der Transporteinrichtung entsprechende Erstreckung aufweist, zumindest abschnittsweise trichterartig ausgebildet ist und/oder mit einem Auslauf in eine Zuführeinrichtung (32) für die Sende- und Empfangseinheit (22, 20) mündet.

6. Vorrichtung nach Anspruch 5, wobei die Zuführeinrichtung (32) eine in ihrer Neigung verstellbare schiefe Ebene aufweist, entlang welcher die Glaspartikel oder Scherben (16) zu der an einem unteren Endabschnitt der Zuführeinrichtung (32) angeordneten Sende- und Empfangseinheit (22, 20) leitbar sind.

7. Vorrichtung nach einem der vorhergehenden Ansprüche 5 oder 6, wobei die Zuführeinrichtung (32) zur Sende- und Empfangseinheit (22, 20) hin verjüngend ausgebildet ist.

8. Vorrichtung nach einem der vorhergehenden Ansprüche, wobei Sende- und Empfangseinheit (22, 20) an zueinander gegenüberliegenden Seitenrändem der Transporteinrichtung (12) oder der Zuführeinrichtung (32) angeordnet sind, sodass die zwischen Sende- und Empfangseinheit (22, 20) verlaufenden Strahlen in der Ebene der Transport- oder Zuführeinrichtung oder jeweils im Wesentlichen parallel hierzu verlaufen.

9. Vorrichtung nach einem der vorhergehenden Ansprüche, wobei die Sende- und/oder die Empfangseinheit (22, 20) in einem gepulsten oder einem Continuous Wave (cw) - Modus betreibbar ist.

10. Vorrichtung nach einem der vorhergehenden Ansprüche, wobei die Transporteinrichtung (12) als ein mit einem Gittergeflecht versehenes Transportband mit einer Vielzahl von Öffnungen ausgebildet ist und mehrere Sende- und Empfangseinheiten (22, 20) paarweise und in Förderrichtung (18) voneinander beabstandet entlang einer Verfahrstrecke angeordnet sind.

11. Vorrichtung nach einem der vorhergehenden Ansprüche, wobei die Empfangseinheit (20) an eine Auswerteeinheit (50) gekoppelt ist, welche zur Erzeugung eines akustisch und/oder optisch wahrnehmbaren Warnsignals ausgebildet ist.

12. Vorrichtung nach einem der vorhergehenden Ansprüche, wobei die Auswerteeinheit (50) an eine Steuereinheit (58) des Durchlaufofens und/oder der Fördereinrichtung (12) gekoppelt ist und zur Notabschaltung von Durchlaufofen und/oder Fördereinrichtung (12) ausgebildet ist.

13. Verfahren zur Detektion von Glasbruch in oder an einem zum Sterilisieren oder Depyrogenisieren von Glasbehältern vorgesehenen Durchlaufofen, wobei der Durchlaufofen zumindest eine Transporteinrichtung (12) für die Glasbehälter (14) aufweist, mittels welcher die Glasbehälter (14) durch den Durchlaufofen hindurchgeführt werden, **dadurch gekennzeichnet, dass** solche eine von der Transporteinrichtung (12) vorgegebene Transportebene verlassenden Glaspartikel oder Glasscherben (16) mittels zwischen einer Sende- und Empfangseinheit (22, 20) propagierender elektromagnetischer Strahlung (24) detektiert werden.

## Claims

1. Device for detecting broken glass in a continuous furnace which is intended to sterilize or depyrogenate glass containers (14) and has a transport device (12) for the glass containers, the detection device comprising at least one transmitting unit (22) and one receiving unit (20), the receiving unit (20) being designed to detect the electromagnetic radiation (24) which can be emitted by the transmitting unit, **characterized in that** the transport device (12) and the transmitting and receiving units (22, 20) are positioned and oriented with respect to one another in such a manner that the glass particles or shards of glass (16) produced by broken glass cross the beam path (24) between the transmitting unit (22) and the receiving unit (20) when leaving or after leaving an essentially horizontal transport plane predefined by the transport device (12), and **in that** the transmitting unit (22) and the receiving unit (20) are arranged beneath the transport device (12).

2. Device according to Claim 1, the beams (24) propagating between the transmitting unit (22) and the receiving unit (20) running essentially perpendicular to the direction of movement of the glass particles or shards of glass (16).

3. Device according to one of the preceding claims, the transmitting unit (22) and the receiving unit (20) being arranged in the region of an end section of the transport device (12), which end section is at the front in the conveying direction (18).

4. Device according to one of the preceding claims, a collecting device (30) for glass particles or shards of glass (16) being arranged beneath and in alignment with a front edge of the transport device (12) in the transport direction.

5. Device according to Claim 4, the collecting device (30) having at least an extent which corresponds to the width of the transport device, having a funnel-like design at least in sections and/or opening, with an outlet, into a feed device (32) for the transmitting and receiving units (22, 20).

6. Device according to Claim 5, the feed device (32) having an inclined plane whose inclination can be adjusted and along which the glass particles or shards (16) can be guided to the transmitting and receiving units (22, 20) arranged on a lower end section of the feed device (32).

7. Device according to either of the preceding Claims 5 and 6, the feed device (32) tapering toward the transmitting and receiving units (22, 20).

8. Device according to one of the preceding claims, the transmitting and receiving units (22, 20) being arranged on opposite side edges of the transport device (12) or the feed device (32), with the result that the beams running between the transmitting and receiving units (22, 20) run in the plane of the transport or feed device or each run essentially parallel thereto.

9. Device according to one of the preceding claims, the transmitting unit (22) and/or the receiving unit (20) being able to be operated in a pulsed or a continuous wave (cw) mode.

10. Device according to one of the preceding claims, the transport device (12) being in the form of a conveyor belt which is provided with a grating mesh and has a multiplicity of openings, and a plurality of transmitting and receiving units (22, 20) being arranged in pairs and at a distance from one another in the conveying direction (18) along a displacement path.

11. Device according to one of the preceding claims, the receiving unit (20) being coupled to an evaluation unit (50) which is designed to generate an acoustically and/or optically perceptible warning signal.

12. Device according to one of the preceding claims, the evaluation unit (50) being coupled to a control unit (58) of the continuous furnace and/or of the conveying device (12) and being designed for the emergency shutdown of the continuous furnace and/or the conveying device (12).

13. Method for detecting broken glass in or on a continuous furnace which is intended to sterilize or depyrogenate glass containers, the continuous furnace having at least one transport device (12) for the glass containers (14), which transport device is used to guide the glass containers (14) through the continuous furnace, **characterized in that** such glass particles or shards of glass (16) leaving a transport plane predefined by the transport device (12) are detected using electromagnetic radiation (24) which propagates between a transmitting unit (22) and a receiving unit (20).

## Revendications

1. Ensemble de détection de la rupture de verre dans un four traversé par des récipients en verre (14) pour y être stérilisés ou dépyrogénisés, le four présentant un dispositif (12) qui transporte les récipients en verre,
l'ensemble de détection comportant au moins une unité d'émission et une unité de réception (22, 20), l'unité de réception (20) étant configurée pour détecter le rayonnement électromagnétique (24) qui peut être émis par l'unité d'émission,
**caractérisé en ce que**
le dispositif de transport (12), l'unité d'émission et l'unité de réception (22, 20) sont positionnés et alignés les uns par rapport aux autres de telle sorte que les particules de verre ou éclats de verre (16) formés suite à la rupture du verre croisent le parcours (24) des rayons qui relie l'unité d'émission à l'unité de réception (22, 20) lorsqu'elles quittent ou après qu'elles ont quitté un plan de transport prédéterminé par le dispositif de transport (12) et s'étendant essentiellement à l'horizontale et
**en ce que** l'unité d'émission (22) et l'unité de réception (20) sont disposées en dessous du dispositif de transport (12).

2. Dispositif selon la revendication 1, dans lequel des rayons (24) qui se propagent entre l'unité d'émission (22) et l'unité de réception (20) s'étendent essentiellement perpendiculairement à la direction de déplacement des particules de verre ou des éclats de verre (16).

3. Dispositif selon l'une des revendications précédentes, dans lequel l'unité d'émission (22) et l'unité de réception (20) sont disposées au niveau d'une partie d'extrémité du dispositif de transport (12) située en avant dans la direction de transport (18).

4. Dispositif selon l'une des revendications précédentes, dans lequel un dispositif de reprise (30) des particules de verre ou des éclats de verre (16) est disposé en dessous du bord du dispositif de transport (12) situé en avant dans la direction de transport et est aligné sur ce bord.

5. Dispositif selon la revendication 4, dans lequel le dispositif de reprise (30) présente au moins une extension qui correspond à la largeur du dispositif de transport, est configuré au moins en partie en entonnoir et/ou débouche par une sortie dans un dispositif d'amenée (32) prévu pour l'unité d'émission et l'unité de réception (22, 20).

6. Dispositif selon la revendication 5, dans lequel le dispositif d'amenée (32) présente un plan oblique dont l'inclinaison peut être ajustée et le long duquel les particules ou éclats de verre (16) peuvent être amenés vers l'unité d'émission et l'unité de réception (22, 20) disposées sur une partie d'extrémité inférieure du dispositif d'amenée (32).

7. Dispositif selon l'une des revendications 5 ou 6 qui précèdent, dans lequel le dispositif d'amenée (32) se rétrécit en direction de l'unité d'émission et de l'unité de réception (22, 20).

8. Dispositif selon l'une des revendications précédentes, dans lequel l'unité d'émission et l'unité de réception (22, 20) sont disposées sur des bords latéraux mutuellement opposés du dispositif de transport (12) ou du dispositif d'amenée (32) de telle sorte que les rayons qui se déplacent entre l'unité d'émission et l'unité de réception (22, 20) s'étendent dans le plan du dispositif de transport ou du dispositif d'amenée, ou essentiellement en parallèle à ces plans.

9. Dispositif selon l'une des revendications précédentes, dans lequel l'unité d'émission et/ou l'unité de réception (22, 20) peuvent fonctionner en mode d'ondes pulsées ou continues (cw).

10. Dispositif selon l'une des revendications précédentes, dans lequel le dispositif de transport (12) est configuré comme bande transporteuse dotée d'un treillis en grille et présentant de nombreuses ouvertures, plusieurs unités d'émission et unités de réception (22, 20) étant disposées par paires et à distance les unes des autres dans la direction de transport (18) le long d'un parcours de déplacement.

11. Dispositif selon l'une des revendications précédentes, dans lequel l'unité de réception (20) est couplée à une unité d'évaluation (50) configurée pour produire un signal d'alarme qui peut être perçu par voie acoustique et/ou optique.

12. Dispositif selon l'une des revendications précédentes, dans lequel l'unité d'évaluation (50) est couplée à une unité de commande (58) du four et/ou du dispositif de transport (12) et est configurée pour débrancher le four et/ou le dispositif de transport (12) en cas d'urgence.

13. Procédé de détection de la rupture du verre dans ou sur un four traversé par des récipients en verre qui y sont stérilisés ou dépyrogénisés,
le four présentant au moins un dispositif de transport (12) des récipients de verre (14) au moyen duquel les récipients de verre (14) sont guidés à travers le four,
**caractérisé en ce que**
ces particules de verre ou éclats de verre (16) qui quittent un plan de transport prédéterminé par le dispositif de transport (12) sont détectés au moyen d'un rayonnement électromagnétique (24) qui se propage entre une unité d'émission et une unité de réception (22, 20).
